(19) 

Europäisches
Patentamt
European
Patent Office
Office européen
des brevets

(11) **EP 2 848 200 A1**

(12) **EUROPEAN PATENT APPLICATION**

(43) Date of publication:
**18.03.2015 Bulletin 2015/12**

(51) Int Cl.:
**A61B 8/08** (2006.01)   **A61B 8/00** (2006.01)
**A61B 8/06** (2006.01)

(21) Application number: **14173024.2**

(22) Date of filing: **18.06.2014**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **13.09.2013 KR 20130110613**

(71) Applicant: **Samsung Medison Co., Ltd.
Gangwon-do 250-870 (KR)**

(72) Inventors:
• **Hyun, Dong-gyu
Gangwon-do (KR)**
• **Choi, Seok-won
Gangwon-do (KR)**

(74) Representative: **Schmid, Wolfgang
Lorenz & Kollegen
Patentanwälte Partnerschaftsgesellschaft mbB
Alte Ulmer Strasse 2
89522 Heidenheim (DE)**

(54) **Method and Apparatus for Providing Ultrasound Information By Using Guidelines**

(57) Disclosed are an ultrasound information providing method and apparatus. The ultrasound information providing method includes generating a Doppler image from Doppler data of an object, acquiring a guideline indicating a moving path of the object in the Doppler image, correcting the Doppler data on the basis of an angle between the guideline and a beam axis of a probe, and displaying a Doppler image generated from the corrected Doppler data.

FIG. 2

EP 2 848 200 A1

**Description**

RELATED APPLICATIONS

This application claims the benefit of Korean Patent Application No. 10-2013-0110613, filed on September 13, 2013, in the Korean Intellectual Property Office, the disclosure of which is incorporated herein in its entirety by reference.

BACKGROUND

1. Field

One or more embodiments of the present invention relate to a method and apparatus for providing information about a diagnosis of an object on a screen by using an ultrasound information providing apparatus, and more particularly, to a method and apparatus for accurately and efficiently displaying a motion of an object by using a guideline of a Doppler image.

2. Description of the Related Art

Ultrasound diagnostic apparatuses transmit an ultrasound signal (generally having a frequency of 20 kHz or more) to an internal part of an object by using a probe, and obtain an image of the internal part of the object by using information of an echo signal reflected from the object. In particular, the ultrasound diagnostic apparatuses are used for medical purposes such as inspecting the inner area of an object, detecting a foreign material in a body, and assessing an injury. The ultrasound diagnostic apparatuses have a higher stability than diagnostic apparatuses using X-rays, display an image in real time, and are safe because there is no exposure to radioactivity, and thus, may be widely used along with other image diagnostic apparatuses.

An image (hereinafter referred to as an ultrasound image) obtained from an ultrasound diagnostic apparatus may be displayed by the ultrasound diagnostic apparatus, or may be stored in a storage medium and displayed by another image display device. For example, a size of an ultrasound image may be reduced by a portable terminal, a portable electronic device, a personal digital assistant (PDA), a tablet personal computer (PC), or the like, and the ultrasound image may be displayed on a screen.

Doppler imaging enables a user to know that an object becomes closer to or farther away from a probe which transmits and receives an ultrasound signal, and thus is mainly used to detect a motion of the object.

SUMMARY

One or more embodiments of the present invention include a method and apparatus for providing ultrasound information about a motion of an object by using guidelines.

One or more embodiments of the present invention include a non-transitory computer-readable storage medium storing a program for executing the method in a computer.

Additional aspects will be set forth in part in the description which follows and, in part, will be apparent from the description, or may be learned by practice of the presented embodiments.

According to one or more embodiments of the present invention, an ultrasound information providing method includes: generating a first Doppler image from Doppler data of an object; acquiring a guideline indicating a moving path of the object in the first Doppler image; correcting the Doppler data on a basis of an angle between the guideline and a beam axis of a probe; and displaying a second Doppler image generated from the corrected Doppler data.

The acquiring may include acquiring the guideline on a basis of a user input, which draws a line on the first Doppler image, or a guideline detecting algorithm.

The ultrasound information providing method may further include displaying the guideline.

The displaying of the guideline may include displaying the guideline on at least one of the first and second Doppler images.

The correcting may include correcting the Doppler data to data previous to projection onto the beam axis by using the angle.

The angle may include an acute angle between the guideline and the beam axis.

The ultrasound information providing method may further include setting a region of which the Doppler data is to be corrected.

The region may include a thickness of the object.

The setting may include setting the region on a basis of a user input or a blood vessel region detecting algorithm.

The correcting may include, when the angle is within a predetermined range, correcting the Doppler data by using spatially near Doppler data.

The ultrasound information providing method may further include, when the corrected Doppler data is not within a range of a color map matched with the Doppler data previous to the correction, scaling up the color map.

The Doppler data may include two-dimensional (2D) Doppler data or three-dimensional (3D) Doppler data.

The Doppler data may include at least one of color Doppler data indicating blood flow and tissue Doppler data indicating a motion of a tissue.

According to one or more embodiments of the present invention, an ultrasound information providing method includes: acquiring a guideline indicating a moving path of an object from Doppler data of the object; correcting the Doppler data on a basis of an angle between the guideline and a beam axis of a probe; and displaying a Doppler image generated from the corrected Doppler data.

According to one or more embodiments of the present invention, an ultrasound information providing apparatus includes: a data acquirer that acquires Doppler data of an object; an image processor that generates a first Doppler image from the Doppler data of the object, and acquires a guideline indicating a moving path of the object in the first Doppler image; a data processor that corrects the Doppler data on a basis of an angle between the guideline and a beam axis of a probe; and a display unit that displays a second Doppler image generated from the corrected Doppler data.

According to one or more embodiments of the present invention, an ultrasound information providing apparatus includes: a data acquirer that acquires Doppler data of an object; an image processor that acquires a guideline indicating a moving path of the object from the Doppler data; a data processor that corrects the Doppler data on a basis of an angle between the guideline and a beam axis of a probe; and a display unit that displays a Doppler image which is generated by the image processor by using the corrected Doppler data.

According to one or more embodiments of the present invention, provided is a non-transitory computer-readable storage medium storing a computer program for executing the ultrasound information providing method.

BRIEF DESCRIPTION OF THE DRAWINGS

These and/or other aspects will become apparent and more readily appreciated from the following description of the embodiments, taken in conjunction with the accompanying drawings in which:

FIG. 1 is a diagram for describing a Doppler angle and a Doppler frequency according to an embodiment of the present invention;

FIG. 2 is a block diagram of an ultrasound information providing apparatus according to an embodiment of the present invention;

FIG. 3 is a flowchart of an ultrasound information providing method according to an embodiment of the present invention;

FIG. 4 is a flowchart of an ultrasound information providing method according to another embodiment of the present invention;

FIG. 5 is a diagram for describing a guideline according to an embodiment of the present invention;

FIG. 6A, FIG. 6B, FIG. 6C, and FIG. 6D are diagrams for describing an operation of automatically detecting a guideline, according to an embodiment of the present invention;

FIG. 7A and FIG. 7B are diagrams for describing an operation of correcting Doppler data by using an angle between a guideline and a beam axis, according to an embodiment of the present invention;

FIG. 8 is a diagram for describing an operation of setting a region of which Doppler data is to be corrected, according to an embodiment of the present invention;

FIG. 9A and FIG. 9B are diagrams for describing an operation of correcting Doppler data by using nearby Doppler data, according to an embodiment of the present invention;

FIG. 10A and FIG. 10B are graphs for describing an operation of scaling up a color map matched with Doppler data, according to an embodiment of the present invention;

FIG. 11 is a diagram for describing an operation of correcting color Doppler data and tissue Doppler data, according to an embodiment of the present invention; and

FIG. 12 is a diagram for describing an operation of correcting three-dimensional (3D) Doppler data, according to an embodiment of the present invention.

DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description.

Expressions such as "at least one of," when preceding a list of elements, modify the entire list of elements and do not modify the individual elements of the list.

The terms used in the present invention have been selected as general terms widely used at present, in consideration of the functions of the present invention, but may be altered according to the intent of an operator skilled in the art, conventional practice, or introduction of new technology. Also, if a term is arbitrarily selected by the applicant in a specific case, a meaning of the term in this specific case will be described in detail in a corresponding description portion of the present invention. Therefore, the terms should be defined on the basis of the entire content of this specification instead of a simple name of each of the terms.

In the disclosure below, when it is described that one device comprises (or includes or has) some elements, it should be understood that the device may comprise (or include or has) only those elements, or it may comprise (or include or have) other elements as well as those elements if there is no specific limitation. Moreover, each term such as "...unit", "...apparatus" and "module" described in specification denotes an element for performing at least one function or operation, and may be implemented via hardware, software, or a combination of hardware and software.

The term "ultrasound image" used herein denotes an image of an object acquired by using an ultrasound wave. Also, the term "object" used herein may include a person, an animal, a part of the person, or a part of the animal. For example, an object may include an organ such as a liver, a heart, a womb, a brain, breasts, an abdomen, or the like, or a blood vessel. Also, the term "object" may include a phantom. The phantom denotes a material having a volume that is very close to a density and effective atomic number of an organism, and may include a spherical phantom having a characteristic similar to a physical body.

Moreover, the term "user" used herein is a medical expert, and may be a doctor, a nurse, a medical technologist, a medical image expert, or the like, or may be an engineer who repairs a medical apparatus. However, the user is not limited thereto.

Hereinafter, embodiments of the present invention will be described in detail with reference to the accompanying drawings.

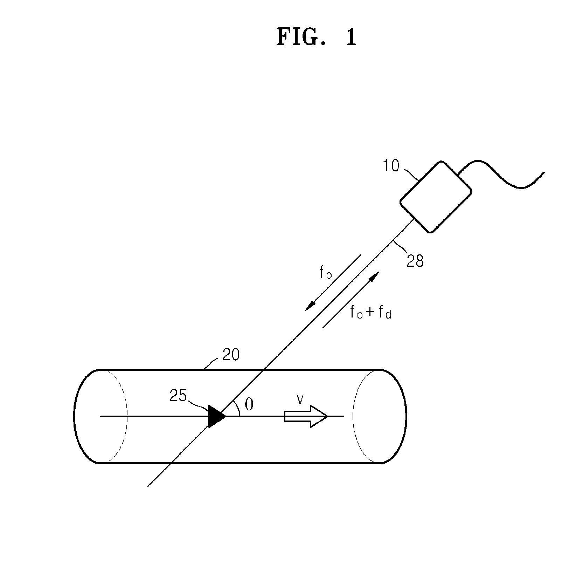

FIG. 1 is a diagram for describing a Doppler angle and a Doppler frequency according to an embodiment of the present invention.

A probe 10 of FIG. 1 transmits an ultrasound signal to an object 25 which moves through a blood vessel 20, and receives an ultrasound signal (an echo signal) reflected from the object 25. The probe 10 transmits the ultrasound signal having a frequency "f0" to the object 25 which moves at a speed "v" in the blood vessel 20. The ultrasound signal transmitted by the probe 10 may focus on the object 25, and the focused ultrasound signal may be expressed as one axis. Hereinafter, a central axis of the ultrasound signal transmitted by the probe 10 is referred to as a beam axis 28.

The object 25 scatters the ultrasound signal transmitted by the probe 10 to generate an echo signal that has a frequency "$f_o+f_d$". Hereinafter, a Doppler frequency denotes a difference frequency "$f_d$" between a frequency of the ultrasound signal transmitted by the probe 10 and a frequency of the echo signal received by the probe 10. That is, the Doppler frequency "$f_d$" may denote a frequency which is Doppler-shifted by the object 25.

A Doppler angle denotes an angle "θ" between the beam axis 28 and a moving direction (for example, a central axis of the blood vessel 20) of the object 25. In this case, the Doppler frequency "$f_d$" may be expressed as the following Equation (1):

$$f_d = \left(2 * f_o * v * \cos\theta\right)/c \qquad \ldots(1)$$

where $f_o$ denotes a frequency of the transmitted ultrasound signal, v denotes a moving speed of the object 25, c denotes a speed of sound, and θ denotes the Doppler angle. That is, the Doppler frequency is relevant to the moving speed and Doppler angle of the object 25 as well as a transmission ultrasound frequency.

The Doppler frequency "$f_d$" is expressed as a component which is obtained by projecting the moving speed of the object 25 onto the beam axis 28. Therefore, a Doppler image obtained by imaging Doppler data may be differently expressed according to the Doppler angle "θ" even when measuring the same object 25.

As the Doppler angle becomes closer to 0, namely, as the moving direction of the object 25 becomes closer to a direction toward the probe 10, the Doppler frequency "$f_d$" expresses the moving speed of the object 25 well. In other words, there are a few errors between a Doppler imaging result using the Doppler data and an actual moving speed of the object 25. On the other hand, as the Doppler angle becomes closer to 90 degrees, cosθ becomes closer to 0, and thus, there are many errors between the Doppler frequency "$f_d$" and an actual speed "v". That is, the actual moving speed of the object 25 is not reflected well in the Doppler data.

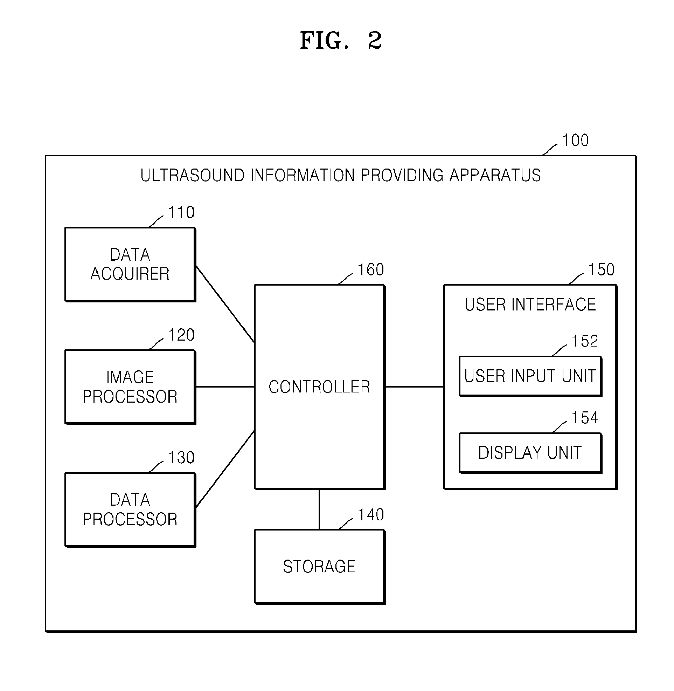

FIG. 2 is a block diagram of an ultrasound information providing apparatus 100 according to an embodiment of the present invention. Referring to FIG. 2, the ultrasound information providing apparatus 100 according to an embodiment may include a data acquirer 110, an image processor 120, a data processor 130, a storage 140, a user interface 150, and a controller 160. The elements included in the ultrasound information providing apparatus 100 are not limited thereto, and the ultrasound information providing apparatus 100 may further include other general-use elements.

The ultrasound information providing apparatus 100 provides an ultrasound image, obtained by scanning an object, to a user. The ultrasound image may include a 3D image as well as a two-dimensional (2D) image indicating a cross-sectional surface of the object, and include a Doppler image that expresses a motion of an object in colors by using Doppler data, in addition to a grayscale ultrasound image that is generated by scanning the object according to an amplitude (A) mode, a brightness (B) mode, and a motion (M) mode.

The ultrasound information providing apparatus 100 may display various kinds of Doppler images to provide ultrasound information to a user. The Doppler image according to an embodiment may include a color Doppler image (also called a blood Doppler image) indicating blood flow, a tissue Doppler image indicating a motion of tissue, and a spectral Doppler image that expresses a moving speed of an object as a waveform.

Although specifically described below, the ultrasound information providing apparatus 100 may provide ultrasound diagnostic information that is generated by directly scanning an object, but may receive ultrasound diagnostic information from an external device or server connected thereto over a network to provide the ultrasound diagnostic information. In the former, the ultrasound information providing apparatus 100 may be implemented as a fixed/mobile ultrasound diagnostic apparatus. In the latter, however, the ultrasound information providing apparatus 100 may be implemented in various types that include a screen for providing ultrasound information.

For example, the ultrasound information providing apparatus 100 may be implemented in various types, which include a screen for providing ultrasound information, such as work stations, picture archiving and communication system (PACS) viewers, portable phones, smartphones, smart televisions (TVs), Internet protocol (IP) TVs, digital TVs, personal computers (PCs), notebook computers, laptop computers, tablet PCs, e-book terminals, personal digital assistants (PDAs), portable multimedia players (PMPs), navigation terminals, consumer electronic (CE) devices, etc.

The data acquirer 110 acquires ultrasound diagnostic information of an object. The ultrasound diagnostic information according to an embodiment may include the above-described Doppler data. The data acquirer 110 may scan the object and process an echo signal by using a probe, thereby acquiring ultrasound diagnostic information. Also, the data acquirer 110 may acquire ultrasound diagnostic information from an external device or server over a network which is connected thereto in a wired manner or a wireless manner.

When the data acquirer 110 scans an object to acquire ultrasound diagnostic information, one or more probes may be used. That is, the data acquirer 110 may acquire ultrasound diagnostic information by using various kinds of probes such as a linear array probe, a convex array probe, a phased array probe, a matrix array probe.

On the other hand, when the data acquirer 110 acquires ultrasound diagnostic information over a network, various types of communication units may be used. For example, the data acquirer 110 may be connected to the network through a wired connection, such as a USB cable, a data cable, or an optical fiber cable, or a wireless connection such as Bluetooth, Wi-Fi, near field communication (NFC), or a 2G/3G/4G network. The data acquirer 110 may acquire ultrasound diagnostic information from an external device, a hospital server, or a cloud server. The data acquirer 110 may acquire data from the other internal device or server of the hospital server connected to a PACS. The ultrasound diagnostic or Doppler data acquired by the data acquirer 110 may be stored in the below-described storage 140.

The Doppler data acquired by the data acquirer 110 may include information about a moving direction and speed of the object. The Doppler data, as described above, may include color Doppler data or tissue Doppler data depending on a kind of the object, and include two kinds of Doppler data.

The Doppler data may include three-dimensional space Doppler (3D Doppler) data in addition to two-dimensional space Doppler (2D Doppler) data depending on a type of an echo signal. Also, the Doppler data may include Doppler data of a continuous image such as a moving image, in addition to Doppler data of a still image.

The image processor 120 processes the ultrasound diagnostic information acquired by the data acquirer 110 to generate an ultrasound image. The image processor 120, as described above, may generate various kinds of ultrasound images such as a grayscale ultrasound image and a Doppler image indicating a motion of an object. The image processor 120 may perform 2D rendering or 3D rendering for providing information on a screen.

The image processor 120 according to an embodiment may match a color map with the Doppler data included in the ultrasound diagnostic information to generate a Doppler image. That is, the image processor 120 may match a color with a component of a moving speed of the object that is included in the Doppler data, and visually provide information of a moving object to a user.

The image processor 120 may acquire a guideline in addition to the above-described ultrasound image, and perform imaging on the guideline. The guideline may denote a moving route of the object which is displayed on the ultrasound image, and the image processor 120 may generate the guideline to be displayed by using a graphical user interface (GUI).

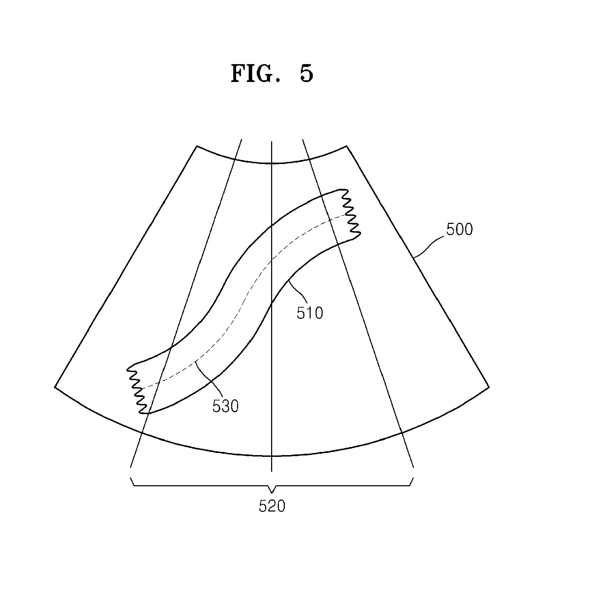

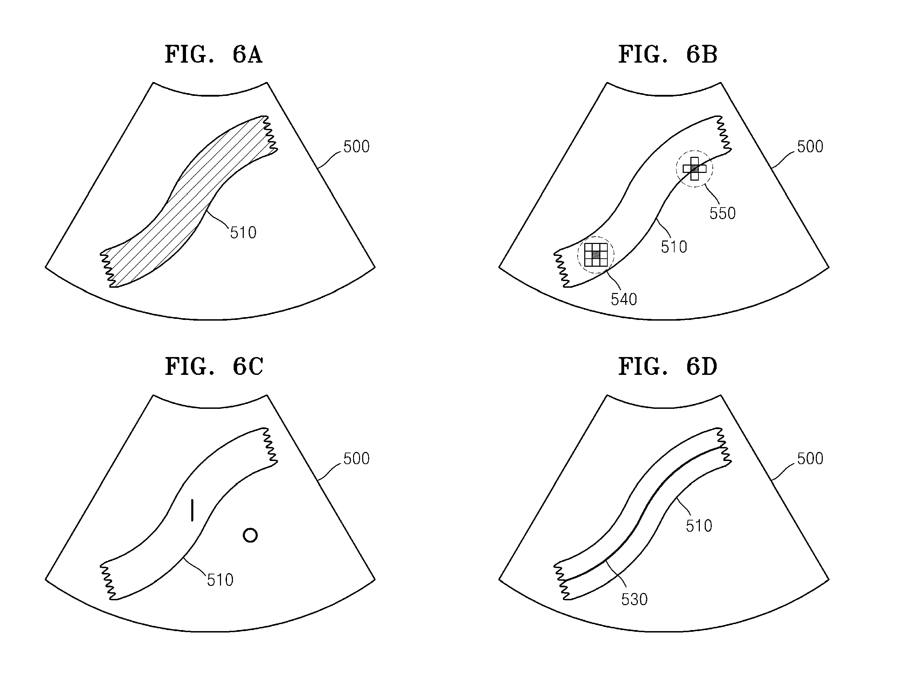

The image processor 120 according to an embodiment may acquire the guideline on the basis of a user input. That is, the image processor 120 may generate the guideline on the basis of a user input that draws a line, such as a straight line, a curve, or the like, on the ultrasound image or the Doppler image. Alternatively, the image processor 120 may generate the guideline on the basis of a pre-stored guideline detecting algorithm. An embodiment, in which the image processor 120 acquires and generates the guideline, will be described in detail with reference to FIGS. 5 and 6.

The image processor 120 may acquire the guideline by using at least one of the Doppler image and the Doppler data. That is, the image processor 120 may acquire the guideline on the basis of a user input for the Doppler image, and may acquire the guideline by applying the guideline detecting algorithm to the Doppler data. Also, the image processor 120 may acquire the guideline by using the Doppler image and the Doppler data.

The image processor 120 may generate a 3D guideline of a 3D image in addition to a guideline of a 2D image. The 3D guideline may denote a straight line or a curve which is displayed on a 3D space.

In addition to the above-described details, the image processor 120 may perform imaging on a variety of information displayed on a screen of the ultrasound information providing apparatus 100. For example, the image processor 120 may generate elements and a variety of information about an ultrasound diagnosis of the object by using a user interface (UI) or a GUI.

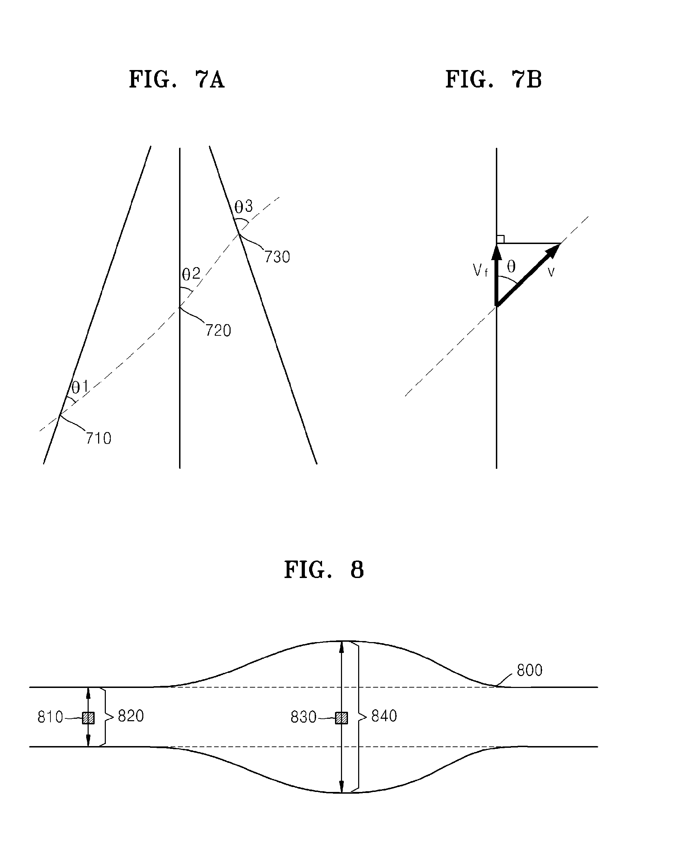

The data processor 130 processes the ultrasound diagnostic information received from the data acquirer 110. The data processor 130 may correct the Doppler data included in the ultrasound diagnostic information. Although specifically described below with reference to FIG. 7A and FIG. 7B, the data processor 130 may correct the Doppler data by using the above-described guideline, and correct the Doppler data on the basis of an angle between the guideline and a beam axis of the probe. The data processor 130 may correct the Doppler data to data previous to projection onto the beam axis of the probe.

The ultrasound diagnostic information (i.e., the corrected Doppler data) obtained through processing by the data processor 130 may be stored in the below-described storage 140, and the image processor 120 may generate an ultrasound image by using the processed ultrasound diagnostic information.

The data processor 130 may set a detailed region, of which ultrasound diagnostic information is to be processed, before processing the ultrasound diagnostic information as described above. For example, the data processor 130 may set a region (i.e., a region of interest (ROI)) of which Doppler data is to be corrected, on the basis of a user input, a blood vessel region detecting algorithm, or a tissue region detecting algorithm. Subsequently, the data processor 130 may correct the Doppler data of the set region in a whole region. The region to be corrected may be a partial or whole region of an object, and for example, when the object is a blood vessel, the region to be corrected may be a thickness of the blood vessel.

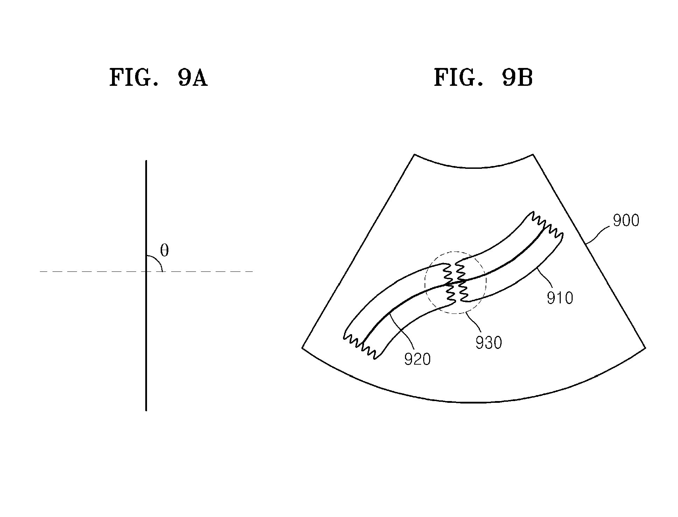

The data processor 130 may use Doppler data at a spatially near position in an operation of correcting specific Doppler data. As described above with reference to FIG. 1, when a Doppler angle is within a certain range, many errors occur in a Doppler imaging result, and thus, the data processor 130 may correct the Doppler data by using information of an ambient space. A detailed embodiment will be described below with reference to FIG. 9A and FIG. 9B.

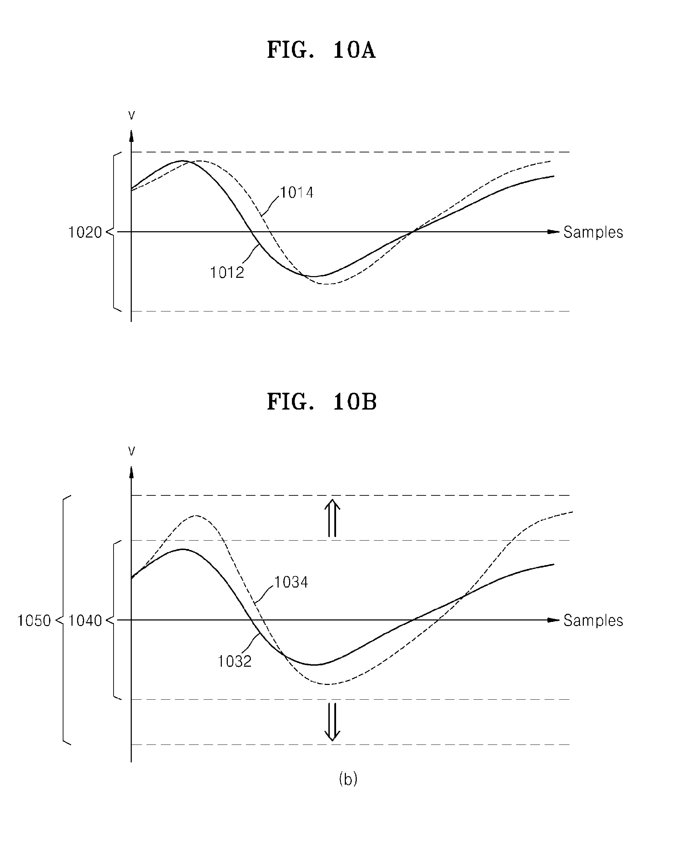

Moreover, the data processor 130 may scale up or down a color map in correcting Doppler data. That is, when the corrected Doppler data is not within a range of the color map matched with Doppler data previous to the correction, the data processor 130 may adjust the color map so as to prevent aliasing from occurring. A detailed embodiment will be described below with reference to FIG. 10A and FIG. 10B.

The storage 140 stores a variety of information obtained through processing by the ultrasound information providing apparatus 100. For example, the storage 140 may store medical data, such as input/output ultrasound data, ultrasound diagnostic information, and ultrasound images, associated with a diagnosis of an object, and may also store an algorithm or a program which is executed in the ultrasound information providing apparatus 100.

The storage 140 may include at least one type of storage medium of a flash memory, a hard disk, a multimedia micro card, a card type memory (a secure digital (SD) card, an extreme digital (XD) card, or the like), random access memory (RAM), static random access memory (SRAM), read-only memory (ROM), electrically erasable programmable read-only memory (EEPROM), and programmable read-only memory (PROM). Also, the ultrasound information providing apparatus 100 may use a web storage or a cloud server which performs a storage function of the storage 140 on the Web.

The user interface 150 provides ultrasound diagnostic information to a user, and receives various kinds of inputs for controlling the ultrasound information providing apparatus 100 from the user. The user interface 150, as illustrated in FIG. 2, may include a user input unit 152 and a display unit 154.

The user input unit 152 receives an external input signal for controlling the ultrasound information providing apparatus 100 from the user. That is, the user input unit 152 may receive a user input through various input units such a keypad, a mouse, a touch panel, a touch screen, a trackball, a jog wheel, a jog switch, etc., and moreover receive a remote input through a remote control unit. For example, the user input unit 152 may receive a user input, which draws a guideline, and a user input that sets a region of which Doppler data is to be corrected.

A touch panel may detect a proximity touch in which a pointer, which is separated from a screen by a certain distance, approaches a position on the screen, in addition to a real touch in which the pointer actually touches a screen. The pointer used herein denotes a touch instrument for really touching or proximity-touching a specific portion of the touch panel. Examples of the pointer include a stylus pen or a body part, such as a finger or the like, of a human body.

Moreover, the touch panel may be implemented as a touch screen which forms a layer structure including the display unit 154. The touch screen may be implemented in various types such as a contact capacitive type, a press resistive type, an infrared sensing type, a surface ultrasonic conductive type, an integration tension measurement type, and a piezo effect type.

Although not shown, the touch panel may include various sensors which are provided inside or near a touch screen, for detecting a touch input. An example of a sensor for sensing a touch of the touch panel is a tactile sensor. The tactile sensor denotes a sensor that senses a touch by a specific object by a degree, at which a user feels, or more. The tactile sensor may sense various pieces of information such as a roughness of a touched surface, a stiffness of a touched object, a temperature of a touched point, etc.

Moreover, an example of a sensor for sensing a touch of the touch panel is a proximity sensor. The proximity sensor denotes a sensor that detects an object approaching a detection surface or an object near the detection surface by using an electromagnetic force or infrared light without mechanical contact. Examples of the proximity sensor include a transmissive photosensor, a directly reflective photosensor, a mirror reflective photosensor, a high frequency oscillation-type proximity sensor, a capacitive proximity sensor, a magnetic proximity sensor, and an infrared proximity sensor.

The display unit 154 may display a GUI associated with a function setting of the ultrasound information providing apparatus 100, or display ultrasound diagnostic information and an ultrasound image, thereby providing visual information to a user. For example, the display unit 154 may display a variety of information, such as a 2D or 3D ultrasound image, a Doppler image, and a guideline, on a screen.

The display unit 154 may include at least one of a liquid crystal display (LCD), a thin film transistor-liquid crystal display, an organic light-emitting diode (OLED) display, a flexible display, and a 3D display. Also, the ultrasound information providing apparatus 100 may include two or more display units 154 depending on an implementation type of the ultrasound information providing apparatus 100.

As described above, the display unit 154 may be configured with the touch screen which forms the layer structure including the user input 152. That is, the display unit 154 may be used as an output unit and an input unit, in which case the display unit 154 may receive a touch input by using a stylus pen or a body part of a human body.

The controller 160 controls an overall operation of the ultrasound information providing apparatus 100. That is, the controller 160 may control operations between the data acquirer 110, the image processor 120, the data processor 130, the storage 140, and the user interface 150 of FIG. 2.

For example, the controller 160 may control the image processor 120 so as to generate a Doppler image from Doppler data acquired by the data acquirer 110. Alternatively, the controller 160 may control the storage 140 so as to store Doppler data obtained through correction by the data processor 130, or control the data processor 130 so as to correct the Doppler data on the basis of a user input received by the user interface 150.

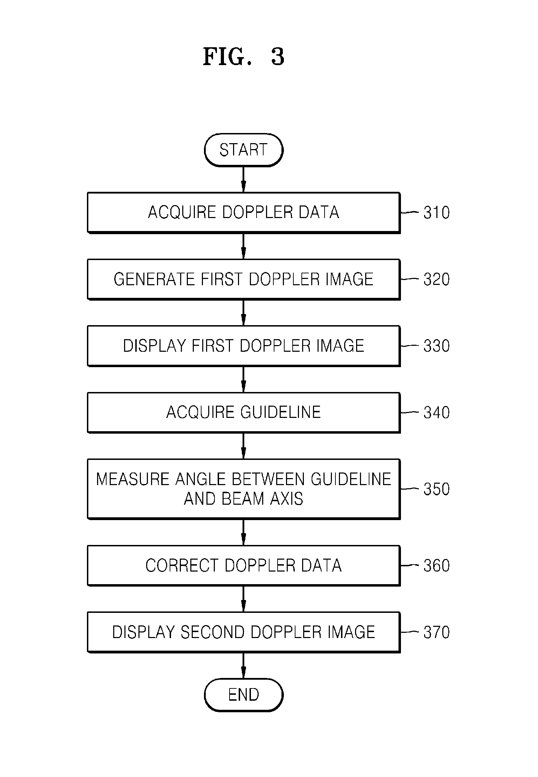

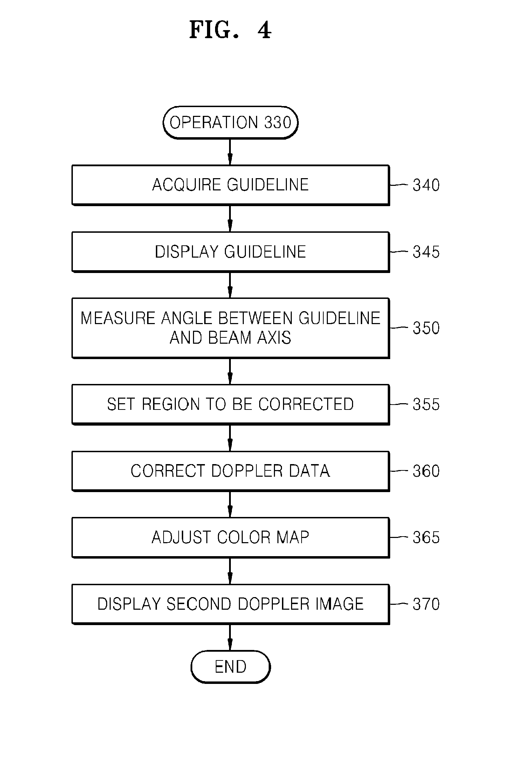

Hereinafter, a method in which the elements included in the ultrasound information providing apparatus 100 provide ultrasound information by using guidelines will be described in detail with reference to FIGS. 3 and 4.

A flowchart of each of FIGS. 3 and 4 includes a plurality of operations that are performed in time series by the data acquirer 110, the image processor 120, the data processor 130, the storage 140, the user interface 150, and the controller 160 of the ultrasound information providing apparatus 100 illustrated in FIG. 2. Thus, although not described below, the above-described details of the elements of FIG. 2 may be applied to the methods of FIGS. 3 and 4.

FIG. 3 is a flowchart of an ultrasound information providing method according to an embodiment of the present invention.

In operation 310, the ultrasound information providing apparatus 100 acquires Doppler data. The ultrasound information providing apparatus 100 may scan an object with a probe to directly acquire the Doppler data, or receive the Doppler data through communication with an external device or server over a network.

As described above, the Doppler data may include 2D or 3D Doppler data, and include at least one of color Doppler data and tissue Doppler data.

In operation 320, the ultrasound information providing apparatus 100 generates a first Doppler image by using the Doppler data which is received in operation 310. The ultrasound information providing apparatus 100 may map a color map to a component of a moving speed of the object that is included in the Doppler data, thereby expressing a motion of the object in colors. In spectral Doppler data, the ultrasound information providing apparatus 100 may express a motion of the object as a waveform.

Subsequently, in operation 330, the ultrasound information providing apparatus 100 displays the first Doppler image which is generated in operation 320.

In operation 340, the ultrasound information providing apparatus 100 acquires a guideline. As described above with reference to FIG. 2, the guideline may denote a moving route of the object in the Doppler image. The guideline according to an embodiment may be disposed in a region which is displayed in colors according to the Doppler data in the Doppler image.

The ultrasound information providing apparatus 100 may sense a user input for the first Doppler image which is displayed in operation 330, and acquire the guideline on the basis of the user input. For example, when the user draws a line on the first Doppler image through a touch screen or a touch panel, the ultrasound information providing apparatus 100 may generate the guideline according to the user input.

Alternatively, the ultrasound information providing apparatus 100 may apply the guideline detecting algorithm to the first Doppler image to automatically generate the guideline. This will be described in detail with reference to FIG. 6A, FIG. 6B, FIG. 6C, and FIG. 6D.

In operation 350, the ultrasound information providing apparatus 100 measures angles between the guideline and beam axes. The ultrasound information providing apparatus 100 measures the angles between the guideline (which is acquired in operation 340) and the beam axes with respect to one position of the first Doppler image. According to an embodiment, the ultrasound information providing apparatus 100 may select and measure an acute angle from the angles between the guideline and the beam axes.

In operation 360, the ultrasound information providing apparatus 100 corrects the Doppler data. The ultrasound information providing apparatus 100 may correct Doppler data at the position, where the angles are measured in operation 350, to data previous to projection according to Equation (1), which has been described above with reference to FIG. 1. Therefore, the ultrasound information providing apparatus 100 may correct the Doppler data to data close to an actual moving speed of the object, and provide information about a diagnosis of the object, thereby reducing an error.

In operation 370, the ultrasound information providing apparatus 100 displays a second Doppler image on the basis of the Doppler data which is obtained through the correction in operation 360. In other words, the ultrasound information providing apparatus 100 may newly generate the second Doppler image by using the corrected Doppler data, and display the second Doppler image. That is, the ultrasound information providing apparatus 100 provides a diagnosis result, in which an influence of the Doppler angle on the Doppler data is minimized, to the user.

FIG. 4 is a flowchart of an ultrasound information providing method according to another embodiment of the present invention. In FIG. 4, an operation subsequent to operations 310 to 330 described above with reference to FIG. 3 will now be described.

In operation 340, the ultrasound information providing apparatus 100 acquires the guideline. As described above with reference to FIG. 3, the ultrasound information providing apparatus 100 may acquire the guide line on the basis of the user input or the guideline detecting algorithm.

In operation 345, the ultrasound information providing apparatus 100 may display the acquired guideline on a screen. In FIG. 3, the acquired guideline is merely used for measuring the angles between the guideline and the beam axes, and is not displayed on the screen. On the other hand, the ultrasound information providing apparatus 100 may display the guideline on the first Doppler image. Therefore, the user of the ultrasound information providing apparatus 100 visually checks the guideline, and easily understands a moving route of the object.

In operation 350, the ultrasound information providing apparatus 100 measures the angles between the guideline and the beam axes. A detailed description on this is as described above with reference to FIG. 3.

In operation 355, the ultrasound information providing apparatus 100 sets a region, of which Doppler data is to be corrected, in the displayed first Doppler image. The ultrasound information providing apparatus 100, as described above with reference to FIG. 2, may set a region of which ultrasound diagnostic information is to be processed, and set the region of which the Doppler data is to be corrected, on the basis of a user input or a blood vessel region detecting algorithm. The region to be corrected may be a partial or whole region of the object, and will be described below with reference to FIG. 8.

In operation 360, the ultrasound information providing apparatus 100 corrects the Doppler data. In addition to the details which have been described in operation 360 of FIG. 3, the ultrasound information providing apparatus 100 may correct the Doppler data of the region which is set in operation 355.

In operation 365, the ultrasound information providing apparatus 100 may adjust a color map matched with the Doppler data and the Doppler image. As described above with reference to FIG. 2, when the corrected Doppler data is not within a range of the color map matched with the Doppler data previous to the correction, the ultrasound information providing apparatus 100 may scale up the color map so as to prevent aliasing from occurring.

In operation 370, the ultrasound information providing apparatus 100 displays the second Doppler image by using the adjusted Doppler data.

FIG. 5 is a diagram for describing a guideline according to an embodiment of the present invention.

A Doppler image 500 of FIG. 5 is a Doppler image of a blood vessel 510, and the ultrasound information providing apparatus 100 displays the Doppler image 500, generated based on the Doppler data, on a screen. Three solid lines across the Doppler image 500 respectively indicate beam axes 520 of a probe that transmits an ultrasound signal to the blood vessel 510. The beam axes 520 of FIG. 5 may have a fan shape along a scan line, or unlike the illustrated details, the beam axes 520 may be disposed in parallel with each other in a rectangular ultrasound image.

The ultrasound information providing apparatus 100 acquires a guideline 530 indicating a moving path of blood flow in the blood vessel 510. As described above, the ultrasound information providing apparatus 100 may acquire and generate the guideline 530 according to a received user input.

For example, when a user draws a line along a central line of the blood vessel 510 via a touch input by using a body part of the user, a mouse, or a trackball, the ultrasound information providing apparatus 100 may generate the guideline 530 according to a sensed position of the user input.

The ultrasound information providing apparatus 100 may display the acquired guideline 530 on the Doppler image 500. In FIG. 5, the guideline 530 is illustrated as a dashed line. However, present embodiment is not limited thereto, and the ultrasound information providing apparatus 100 may display the guideline 530 as various line types such as a dotted line, a dashed line, a solid line, or a double line.

On the other hand, the ultrasound information providing apparatus 100 may internally use the acquired guideline 530 in a system without displaying the acquired guideline 530 on the Doppler image 500. The ultrasound information providing apparatus 100, as described above, corrects the Doppler data by using angles between the guideline 530 and the beam axes 520. That is, the ultrasound information providing apparatus 100 may internally use the guideline 530 in the system so as to measure an angle, without displaying the guideline 530 on a screen.

FIG. 6A, FIG. 6B, FIG. 6C, and FIG. 6D are diagrams for describing an operation of automatically detecting a guideline, according to an embodiment of the present invention. The ultrasound information providing apparatus 100, as described above with reference to FIG. 5, may acquire a guideline on the basis of a user input, but may acquire the guideline through a guideline detecting algorithm. Hereinafter, the guideline detecting algorithm will be described with reference to FIGS. 6A to 6D.

In FIG. 6A, a region 510, which is darkly displayed on a Doppler image, is a region including Doppler data which indicates a motion of an object. In FIGS. 6B to 6D, an operation of classifying the region 510 from the Doppler image 500 will be described.

In FIG. 6B, the ultrasound information providing apparatus 100 analyzes the Doppler data on the basis of a connected component analysis (CCA) algorithm. That is, the ultrasound information providing apparatus 100 detects the region 510 from the Doppler image 500 according to a plurality of spatially near pixels including Doppler data, in a pixel or a pixel group having a certain size.

As illustrated in FIG. 6B, the ultrasound information providing apparatus 100 applies an 8-CCA algorithm to a pixel group 540. That is, the ultrasound information providing apparatus 100 may analyze eight pixels disposed close to a top, bottom, left, and right of a pixel, which is darkly displayed at the center, and four-direction diagonal lines in the pixel group 540, and determine whether the pixel group 540 includes Doppler data. The ultrasound information providing apparatus 100 applies a 4-CCA algorithm to a pixel group 550. That is, the ultrasound information providing apparatus 100 may analyze four pixels close to an up direction, down direction, left direction, and right direction of a pixel, which is darkly displayed at the center in the pixel group 550.

Therefore, the ultrasound information providing apparatus 100 may detect a boundary of a region including the Doppler data in the Doppler image 500. Subsequently, the ultrasound information providing apparatus 100 may separate the region including the Doppler data from a region including no Doppler data to detect the region 510.

In FIG. 6C, the ultrasound information providing apparatus 100 displays the analyzed result of the Doppler image 500 as a value of 1 and a value of 0. By using the result analyzed with a CCA algorithm in FIG. 6B, the ultrasound information providing apparatus 100 may store a value of 1 for the region 510 including the Doppler data, and store a value of 0 for the other regions. That is, the ultrasound information providing apparatus 100 may perform a masking operation on the Doppler image 500 to detect a region including information about a motion of an object.

In FIG. 6D, when a region including the Doppler data is detected, the ultrasound information providing apparatus 100 may acquire a guideline 530 through a skeletonization algorithm. The skeletonization algorithm may denote an algorithm that reduces a thickness of a figure so as to extract a shape of a line, having a thickness equal to or less than a certain thickness, from the figure.

The ultrasound information providing apparatus 100 may acquire the guideline 530 through a morphology operation algorithm, in addition to the skeletonization algorithm. That is, when the region 510 is detected, the ultrasound information providing apparatus 100 may detect the guideline 530 by repeatedly applying at least one of a dilation algorithm that enlarges the region 510 and an erosion algorithm that reduces the region 510.

The above-described details of the guideline detecting algorithm that detects the guideline 530 of the region 510 are merely related to an embodiment, but the present embodiment is not limited thereto. The ultrasound information providing apparatus 100 may detect the guideline 530 on the basis of various schemes. The ultrasound information providing apparatus 100 according to an embodiment may detect the longest path included in the region 510 including the Doppler data, and acquire the longest path as the guideline 530.

FIG. 7A and FIG. 7B are diagrams for describing an operation of correcting Doppler data by using an angle between a guideline and a beam axis, according to an embodiment of the present invention.

In FIG. 7A, three height-direction solid lines indicate beam axes of the probe, as described above with reference to FIG. 5, and a width-direction dashed line indicates a guideline. In FIG. 7A, the ultrasound information providing apparatus 100 measures angles between the guideline and the beam axes. The ultrasound information providing apparatus 100 may measure the angles (which are formed along the guideline) between the guideline and the beam axes in a region including Doppler data. The ultrasound information providing apparatus 100 may select an acute angle from among the angles between the guideline and the beam axes.

In FIG. 7A, an embodiment in which the angles between the guideline and the beam axes are respectively measured at positions 710, 720 and 730 is illustrated. The ultrasound information providing apparatus 100 may acquire information about the beam axes from information of an ultrasound signal transmitted by the probe. Alternatively, the ultrasound information providing apparatus 100 may acquire beam-axis information about a Doppler image from beam-axis information about ultrasound data previous to scan conversion.

In FIG. 7B, when the angles between the beam axes and the guideline are measured, the ultrasound information providing apparatus 100 corrects Doppler data at a position, where an angle is measured, by using the measured angles. The ultrasound information providing apparatus 100 may correct Doppler data previous to projection of Doppler data onto the beam axes, by using the angles.

To provide a detailed description, as described above with reference to FIG. 1, when an actual moving speed of the object is "v" in FIG. 7B, the moving speed of the object that is included in the Doppler data is shown as "$v_f$" that is a component projected onto a corresponding beam axis according to a Doppler angle.

The following Equation (2) may be deduced from the above-described Equation (1). Each term of Equation (2) denotes the same term as that of Equation (1).

$$v = \frac{c * f_d}{2 * f_o * \cos \theta} \qquad \dots (2)$$

The ultrasound information providing apparatus 100 may acquire the actual moving speed "v" of the object by using the transmission frequency, the Doppler frequency, and the Doppler angle "θ". Since the guideline is a line indicating a moving path of the object, the ultrasound information providing apparatus 100 may use the angles which are measured in FIG. 7A, instead of the Doppler angle of Equation (2). That is, the ultrasound information providing apparatus 100 may measure the moving speed "v" of the object by using the angles (which are measured in FIG. 7A) between the guideline and the beam axes.

The ultrasound information providing apparatus 100 may correct "$v_f$", which is a component projected onto a corresponding beam axis, to "v" which is measured by using the angles between the guideline and the beam axes, in Doppler data. Subsequently, the ultrasound information providing apparatus 100 may generate and output a Doppler image by using the corrected Doppler data, thus accurately providing information about a motion of the object to a user.

The ultrasound information providing apparatus 100 may display a Doppler image based on the corrected Doppler data, and newly display the guideline on the Doppler image.

FIG. 8 is a diagram for describing an operation of setting a region of which Doppler data is to be corrected, according to an embodiment of the present invention. A solid line of FIG. 8 indicates a blood vessel 800.

The ultrasound information providing apparatus 100 may set a region, of which Doppler data is to be corrected, in a region including Doppler data. As described above in FIG. 2, the ultrasound information providing apparatus 100 may set a region to be corrected on the basis of a user input or a blood vessel region detecting algorithm. The region to be corrected may include a blood vessel or a thickness of tissue which is an object.

To describe FIG. 8 as an example, the ultrasound information providing apparatus 100 may determine a region to be corrected as a thickness 820, for a position 810. That is, the ultrasound information providing apparatus 100 may select a total thickness of the blood vessel 800, which is the region including the Doppler data, as the region to be corrected.

According to an embodiment, the ultrasound information providing apparatus 100 may determine a region to be corrected according to a user input that selects the thickness 820. That is, the ultrasound information providing apparatus 100 may receive a user input that selects a boundary or thickness of the blood vessel 800, and determine a region to be corrected according to the received user input.

According to another embodiment, the ultrasound information providing apparatus 100 may detect the thickness 820 of the blood vessel 800 on the basis of a blood vessel region detecting algorithm, and determine the detected thickness 820 as the region to be corrected. For example, the algorithm that detects the region 510 described above with reference to FIG. 6A, FIG. 6B, FIG. 6C, and FIG. 6D may be used as the blood vessel region detecting algorithm.

Next, an embodiment in which the ultrasound information providing apparatus 100 corrects Doppler data according to a preset region will be described. First, the ultrasound information providing apparatus 100 corrects Doppler data at the position 810 by using an angle between a guideline and a beam axis for the position 810. Then, the ultrasound information providing apparatus 100 may apply the measured angle at the position 810 to all Doppler data of the thickness 820 of the blood vessel 800 to correct the Doppler data. That is, the ultrasound information providing apparatus 100 may apply the Doppler data at the position 810 to the Doppler data of the thickness 820.

Similarly, at a position 830, the ultrasound information providing apparatus 100 may apply an angle (which is measured at the position 830) between a guideline and a beam axis to Doppler data of a thickness 840.

As illustrated in FIG. 8, another embodiment may be applied at the position 830. That is, at the position 830, the blood vessel 800 is thicker than the position 810, and thus, the ultrasound information providing apparatus 100 may apply the angle measured at the position 830 to only the thickness 820 set at the position 810 without applying the angle measured at the position 830 to the thickness 840. In other words, the ultrasound information providing apparatus 100 may arbitrarily set a region to be corrected, and correct Doppler data with the set region being maintained. However, since the thickness of the blood vessel 800 (an object) changes, the ultrasound information providing apparatus 100 may automatically change a region to be corrected, and correct Doppler data.

FIG. 9A and FIG. 9B are diagrams for describing an operation of correcting Doppler data by using nearby Doppler data, according to an embodiment of the present invention. As described above with reference to FIG. 1, as a Doppler angle becomes closer to 90 degrees, a cosine function result of the Doppler angle becomes close to 0, causing a reduction in reliability of Doppler data. Therefore, when a measured angle between a guideline and a beam axis is within a predetermined range, the ultrasound information providing apparatus 100 may use Doppler data at a position which is spatially close to a measured position.

As illustrated in FIG. 9A, the ultrasound information providing apparatus 100 may previously determine a certain range, which enables data of an ambient space to be used, with respect to an angle between a guideline illustrated as a dashed line and a beam axis illustrated as a solid line. According to an embodiment, the ultrasound information providing apparatus 100 may set the certain range to an angle of 85 degrees to less than 95 degrees.

In FIG. 9B, a guideline 920 of a blood vessel 910 is displayed on a Doppler image 900. A Doppler angle of a region 930 is close to 90 degrees, and thus, the blood vessel 910 is illustrated as being discontinuous on the Doppler image 900. The ultrasound information providing apparatus 100 may measure an angle between the guideline and the beam axis in the region 930, and determine whether the measured angle is within the range set in FIG. 9A.

When the angle measured in the region 930 is within the previously determined range, by using Doppler data at a position which is spatially close to a position where the angle within the previously determined range is measured, the ultrasound information providing apparatus 100 may correct Doppler data at the position where the angle is measured. For example, the ultrasound information providing apparatus 100 may use Doppler data at a position previous or subsequent to the position where the angle within the previously determined range is measured, where angles are measured in a region including the Doppler data. The ultrasound information providing apparatus 100 may correct the Doppler data at the position where the angle within the previously determined range is measured, by using ambient Doppler data according to an interpolation technique.

FIG. 10A and FIG. 10B are graphs for describing an operation of scaling up a color map matched with Doppler data, according to an embodiment of the present invention. In the graphs of FIGS. 10A and 10B, the abscissa axis indicates samples of corrected Doppler data, and the ordinate axis indicates a moving speed of an object. Also, a waveform illustrated as a solid line illustrates a moving speed of the object included in Doppler data previous to correction, and a waveform illustrated as a dashed line illustrates a moving speed of the object included in corrected Doppler data, namely, a moving speed previous to projection onto a beam axis.

In FIG. 10A, a moving speed of the object that is illustrated as a waveform 1012 is corrected by using an angle between a guideline and a beam axis for each of the samples of the Doppler data. The moving speed of the object that is included in the corrected Doppler data is illustrated as a waveform 1014.

An upper limit line and lower limit line of FIG. 10A indicate a range of a color map which is used to generate a Doppler image by using the Doppler data. That is, in the color map, the moving speed of the object is matched in a color. In a color map according to an embodiment of FIG. 10A, a speed within a range 1020 may be matched in a color.

In FIG. 10B, the ultrasound information providing apparatus 100 corrects a moving speed of the object that is illustrated as a waveform 1032 to a waveform 1034. The color map, matched for the Doppler data previous to correction, is matched for a speed within a range 1040. Therefore, the waveform 1034 indicating a moving speed subsequent to correction is not within a range that enables the matched color map to be applied, the waveform 1034 may be expressed in an undesired color due to aliasing, which occurs in a Doppler image.

Therefore, when the Doppler data subsequent to correction is not within a color range of the color map matched with the Doppler data previous to correction, the ultrasound information providing apparatus 100 may scale up the range of the color map from the range 1040 to the range 1050, thereby enabling the scaled-up range to cover the moving speed that is illustrated as the corrected waveform 1034.

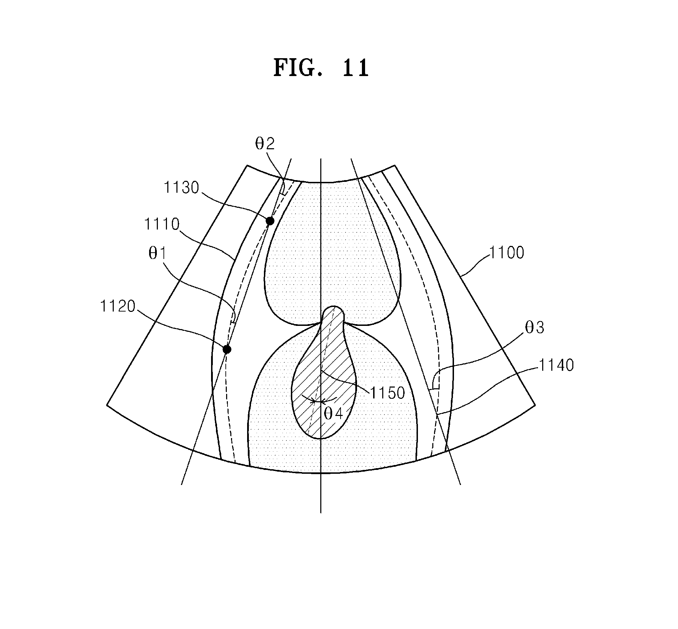

FIG. 11 is a diagram for describing an operation of correcting color Doppler data and tissue Doppler data, according to an embodiment of the present invention. FIG. 11 illustrates a Doppler image 1100 of a heart 1110. In darkly illustrated parts of FIG. 11, an upper part indicates a left atrium (LA), a lower part indicates a left ventricle (LV), and a part connecting the LA and the LV indicates a mitral valve (MV). A part illustrated as an oblique line indicates blood which flows to the LV when the LA contracts. A part illustrated white in the heart 1110 indicates a myocardium surrounding the heart 1110.

The ultrasound information providing apparatus 100, as illustrated, may display at least one of a color Doppler image and a tissue Doppler image on a screen. That is, the ultrasound information providing apparatus 100 may simultaneously generate the color Doppler image and the tissue Doppler image, and display the color Doppler image and the tissue Doppler image on the screen. For example, the ultrasound information providing apparatus 100 may display the tissue Doppler image obtained by processing tissue Doppler data for the myocardium, and display the color Doppler image obtained by processing color Doppler data for blood flowing to the LV.

As described above, the ultrasound information providing apparatus 100 may correct the color Doppler data by using an angle "$\theta4$" between a guideline and a beam axis at a position 1150, for blood.

The ultrasound information providing apparatus 100 may correct the tissue Doppler data for the myocardium through an operation similar to that of the blood. That is, the ultrasound information providing apparatus 100 acquires a guideline for the myocardium on the basis of a user input or a guideline detecting algorithm. Then, the ultrasound information providing apparatus 100 measures an angle "$\theta1$" between a guideline and a beam axis at a position 1120, an angle "$\theta2$" between a guideline and the beam axis at a position 1130, and an angle "$\theta3$" between a guideline and a beam axis at a position 1140. The ultrasound information providing apparatus 100 may correct the tissue Doppler data to a value previous to projection onto the beam axis, on the basis of each of the angles, and display a new tissue Doppler image based on the corrected Doppler data on a screen.

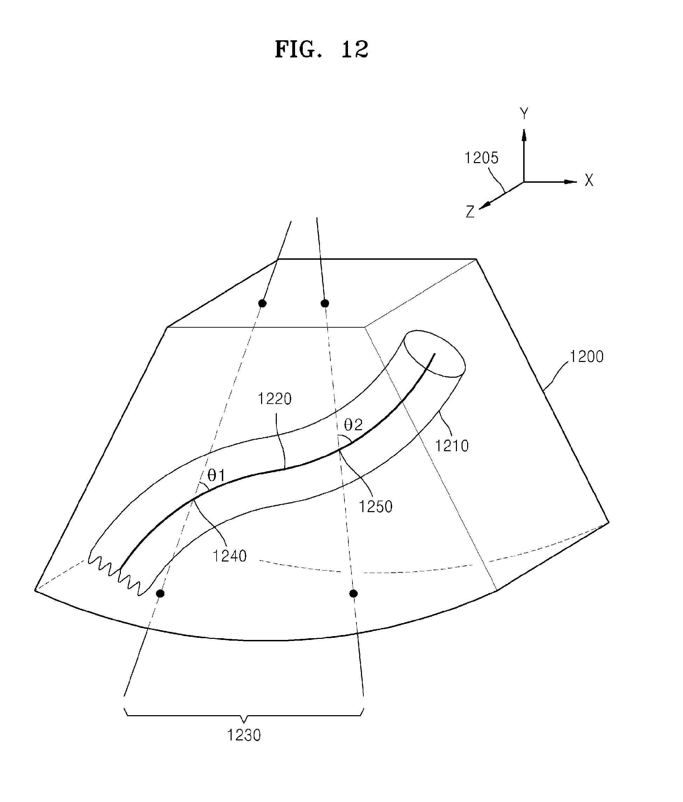

FIG. 12 is a diagram for describing an operation of correcting 3D Doppler data, according to an embodiment of the present invention. A 3D Doppler image 1200 of FIG. 12 displays a blood vessel 1210 disposed in a space by using 3D coordinates 1205.

The ultrasound information providing apparatus 100, as described above, may acquire a guideline 1220 of the blood vessel 1210 displayed on the 3D Doppler image 1200 according to a user input or the guideline detecting algorithm. The ultrasound information providing apparatus 100 may display the generated guideline 1220 on the 3D Doppler image 1200, or may internally use the guideline 1220 in the system without visually displaying the guideline 1220. The guideline 1220 of the 3D Doppler image 1200 may be a straight line or a curve disposed in a 3D space.

The ultrasound information providing apparatus 100 measures an angle "$\theta1$" between the guideline 1220 and a beam axis 1230 of a probe at a position 1240 and an angle "$\theta2$" between the guideline 1220 and the beam axis 1230 at a position 1250. The ultrasound information providing apparatus 100 may correct 3D Doppler data on the basis of the measured angles, and display a 3D Doppler image based on the corrected 3D Doppler data on a screen.

As described above, according to the one or more of the above embodiments of the present invention, the ultrasound information providing apparatus and method minimize errors in a moving speed of an object and Doppler data generated according to a Doppler angle. Therefore, a user accurately diagnoses the object, and efficiently reads out a Doppler image. In addition, the ultrasound information providing apparatus and method minimize a degree by which a result of a Doppler image is changed depending on a user's workmanship, thus enhancing accessibility to the Doppler image.

The above-described method may be written as computer programs and may be implemented in general-use digital computers that execute the programs using computer-readable recording media. The information used in the aforementioned embodiments may be recorded in computer-readable recording media through various members. Program storage devices usable for describing a storage device including executable computer code for performing various methods of the present invention should not be understood as including transitory targets like signals. Examples of the computer-readable recording medium include magnetic storage media (e.g., ROM, floppy disks, hard disks, etc.) and optical recording media (e.g., CD-ROMs or DVDs).

It should be understood that the exemplary embodiments described therein should be considered in a descriptive sense only and not for purposes of limitation. Descriptions of features or aspects within each embodiment should typically be considered as available for other similar features or aspects in other embodiments.

While one or more embodiments of the present invention have been described with reference to the figures, it will be understood by those of ordinary skill in the art that various changes in form and details may be made therein without departing from the spirit and scope of the present invention as defined by the following claims.

**Claims**

**1.** An ultrasound information providing method comprising:

generating a first Doppler image from Doppler data of an object;
acquiring a guideline indicating a moving path of the object in the first Doppler image;
correcting the Doppler data on a basis of an angle between the guideline and a beam axis of a probe; and
displaying a second Doppler image generated from the corrected Doppler data.

**2.** The ultrasound information providing method of claim 1, wherein the acquiring comprises acquiring the guideline on a basis of a user input, which draws a line on the first Doppler image, or a guideline detecting algorithm.

**3.** The ultrasound information providing method of claim 1, further comprising displaying the guideline.

**4.** The ultrasound information providing method of claim 3, wherein the displaying of the guideline comprises displaying the guideline on at least one of the first and second Doppler images.

**5.** The ultrasound information providing method of claim 1, wherein the correcting comprises correcting the Doppler data to data previous to projection onto the beam axis by using the angle.

**6.** The ultrasound information providing method of claim 1, wherein the angle comprises an acute angle between the guideline and the beam axis.

**7.** The ultrasound information providing method of claim 1, further comprising setting a region of which the Doppler data is to be corrected.

**8.** The ultrasound information providing method of claim 7, wherein the region comprises a thickness of the object.

**9.** The ultrasound information providing method of claim 7, wherein the setting comprises setting the region on a basis of a user input or a blood vessel region detecting algorithm.

**10.** The ultrasound information providing method of claim 1, wherein the correcting comprises, when the angle is within a predetermined range, correcting the Doppler data by using spatially near Doppler data.

**11.** The ultrasound information providing method of claim 1, further comprising, when the corrected Doppler data is not within a range of a color map matched with the Doppler data previous to the correction, scaling up the color map.

**12.** The ultrasound information providing method of claim 1, wherein the Doppler data comprises two-dimensional (2D) Doppler data or three-dimensional (3D) Doppler data.

**13.** The ultrasound information providing method of claim 1, wherein the Doppler data comprises at least one of color Doppler data indicating blood flow and tissue Doppler data indicating a motion of tissue.

**14.** An ultrasound information providing apparatus comprising:

a data acquirer that acquires Doppler data of an object;
an image processor that generates a first Doppler image from the Doppler data of the object, and acquires a guideline indicating a moving path of the object in the first Doppler image;
a data processor that corrects the Doppler data on a basis of an angle between the guideline and a beam axis of a probe; and
a display unit that displays a second Doppler image by using the corrected Doppler data.

**15.** The ultrasound information providing apparatus of claim 14, wherein the image processor acquires the guideline on a basis of a user input, which draws a line on the first Doppler image, or a guideline detecting algorithm.

# FIG. 1

FIG. 2

ULTRASOUND INFORMATION PROVIDING APPARATUS — 100

DATA ACQUIRER — 110

IMAGE PROCESSOR — 120

DATA PROCESSOR — 130

CONTROLLER — 160

STORAGE — 140

USER INTERFACE — 150

USER INPUT UNIT — 152

DISPLAY UNIT — 154

# FIG. 3

START

| | |
|---|---|
| ACQUIRE DOPPLER DATA | — 310 |

| | |
|---|---|
| GENERATE FIRST DOPPLER IMAGE | — 320 |

| | |
|---|---|
| DISPLAY FIRST DOPPLER IMAGE | — 330 |

| | |
|---|---|
| ACQUIRE GUIDELINE | — 340 |

| | |
|---|---|
| MEASURE ANGLE BETWEEN GUIDELINE AND BEAM AXIS | — 350 |

| | |
|---|---|
| CORRECT DOPPLER DATA | — 360 |

| | |
|---|---|
| DISPLAY SECOND DOPPLER IMAGE | — 370 |

END

# FIG. 4

```
      ┌─────────────────┐
      │ OPERATION 330   │
      └─────────────────┘
               │
               ▼
   ┌────────────────────────┐
   │   ACQUIRE GUIDELINE     │─── 340
   └────────────────────────┘
               │
               ▼
   ┌────────────────────────┐
   │   DISPLAY GUIDELINE     │─── 345
   └────────────────────────┘
               │
               ▼
   ┌────────────────────────────┐
   │ MEASURE ANGLE BETWEEN      │
   │ GUIDELINE AND BEAM AXIS    │─── 350
   └────────────────────────────┘
               │
               ▼
   ┌────────────────────────┐
   │ SET REGION TO BE        │─── 355
   │ CORRECTED               │
   └────────────────────────┘
               │
               ▼
   ┌────────────────────────┐
   │  CORRECT DOPPLER DATA   │─── 360
   └────────────────────────┘
               │
               ▼
   ┌────────────────────────┐
   │    ADJUST COLOR MAP     │─── 365
   └────────────────────────┘
               │
               ▼
   ┌────────────────────────────┐
   │ DISPLAY SECOND DOPPLER     │─── 370
   │ IMAGE                      │
   └────────────────────────────┘
               │
               ▼
         ┌──────────┐
         │   END    │
         └──────────┘
```

FIG. 5

FIG. 6A

FIG. 6B

FIG. 6C

FIG. 6D

FIG. 7A

FIG. 7B

θ3
730
θ2
720
θ1
710

$V_f$  θ  v

FIG. 8

800
810 — 820
830 — 840

FIG. 9A

FIG. 9B

## FIG. 10A

## FIG. 10B

(b)

# FIG. 11

# FIG. 12

## EUROPEAN SEARCH REPORT

Application Number

EP 14 17 3024

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 701 898 A (ADAM DAN R [IL] ET AL) 30 December 1997 (1997-12-30) * abstract; claims 1-20 * * column 1, line 62 - line 67; figures 1-3 * * column 2, line 12 - line 45 * * column 3, line 38 - line 44 * * column 5, line 47 - line 57 * * page 6, line 35 - line 41 * * column 7, line 1 - line 67 * * column 8, line 16 - column 9, line 19 * | 1-6, 12-15 | INV. A61B8/08 A61B8/00 A61B8/06 |
| X | US 2005/124885 A1 (ABEND KENNETH [US] ET AL) 9 June 2005 (2005-06-09) * abstract; figures 1-5,10-14 * * paragraphs [0006], [0008], [0009], [0027] - [0032], [0044] - [0046] * | 1-6, 12-15 | |
| X | WO 91/11146 A1 (COMMW SCIENT IND RES ORG [AU]) 8 August 1991 (1991-08-08) * abstract; figures 1-4 * * page 7, line 4 - page 8, line 19 * * page 14, line 4 - page 15, line 25 * * page 18, line 22 - page 19, line 22 * * page 20, line 19 - page 22, line 25 * * page 23, line 18 - page 24, line 3 * | 1,2,5-9, 11,14,15 | TECHNICAL FIELDS SEARCHED (IPC) A61B G01S |
| X | US 2003/083578 A1 (ABE YASUHIKO [JP] ET AL) 1 May 2003 (2003-05-01) * paragraphs [0088] - [0089] * | 1,2, 12-15 | |
| X | US 2009/292208 A1 (JEFFREY JR R BROOKE [US] ET AL) 26 November 2009 (2009-11-26) * paragraphs [0010], [0024] - [0027]; figures 3-5 * | 1-6,10, 12-15 | |

-/--

The present search report has been drawn up for all claims

| Place of search | Date of completion of the search | Examiner |
|---|---|---|
| Munich | 6 February 2015 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 14 17 3024

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 8 047 991 B2 (SRINIVASAN SESHADRI [US] ET AL) 1 November 2011 (2011-11-01) * abstract; figures 1-2 * * column 2, line 25 - column 6, line 65 * ----- | 1-6, 12-15 | |
| A | BONNEFOUS O ED - MCAVOY B R: "MEASUREMENT OF THE COMPLETE (3D) VELOCITY VECTOR OF BLOOD FLOWS", PROCEEDINGS OF THE ULTRASONICS SYMPOSIUM. CHICAGO, OCT. 2 - 5, 1988; [PROCEEDINGS OF THE ULTRASONICS SYMPOSIUM], NEW YORK, IEEE, US, vol. 1, 2 October 1988 (1988-10-02), pages 795-799, XP000077044, * page 795, left-hand column, paragraph 4 - paragraph 6; figure 2 * * page 798, left-hand column, paragraph 2 - right-hand column, paragraph 1; figure 5 * ----- | 10 | |
| A | US 2011/196237 A1 (PELISSIER LAURENT [CA] ET AL) 11 August 2011 (2011-08-11) * paragraphs [0004] - [0006], [0115] - [0116]; figure 8 * ----- | 10 | TECHNICAL FIELDS SEARCHED (IPC) |

The present search report has been drawn up for all claims

| Place of search | Date of completion of the search | Examiner |
|---|---|---|
| Munich | 6 February 2015 | Daoukou, Eleni |

EPO FORM 1503 03.82 (P04C01)

**ANNEX TO THE EUROPEAN SEARCH REPORT
ON EUROPEAN PATENT APPLICATION NO.**

EP 14 17 3024

This annex lists the patent family members relating to the patent documents cited in the above-mentioned European search report.
The members are as contained in the European Patent Office EDP file on
The European Patent Office is in no way liable for these particulars which are merely given for the purpose of information.

06-02-2015

| Patent document cited in search report | | Publication date | Patent family member(s) | | Publication date |
|---|---|---|---|---|---|
| US 5701898 | A | 30-12-1997 | NONE | | |
| US 2005124885 | A1 | 09-06-2005 | CA | 2543077 A1 | 12-05-2005 |
| | | | EP | 1682006 A2 | 26-07-2006 |
| | | | JP | 2007509722 A | 19-04-2007 |
| | | | US | 2005124885 A1 | 09-06-2005 |
| | | | WO | 2005041759 A2 | 12-05-2005 |
| WO 9111146 | A1 | 08-08-1991 | JP | H05506371 A | 22-09-1993 |
| | | | US | 5280787 A | 25-01-1994 |
| | | | WO | 9111146 A1 | 08-08-1991 |
| US 2003083578 | A1 | 01-05-2003 | JP | 4734395 B2 | 27-07-2011 |
| | | | JP | 2009011861 A | 22-01-2009 |
| | | | US | 2003083578 A1 | 01-05-2003 |
| US 2009292208 | A1 | 26-11-2009 | NONE | | |
| US 8047991 | B2 | 01-11-2011 | NONE | | |
| US 2011196237 | A1 | 11-08-2011 | NONE | | |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020130110613